# EUROPEAN PATENT APPLICATION

(11) **EP 4 643 655 A1**
(43) Date of publication of application: **05.11.2025**
(21) Application number: 24382471.1
(22) Date of filing: 30.04.2024
(51) Int. Cl.: A23L 3/3508, A23L 27/00, A23L 27/10, A23L 27/24, A23L 27/40, A23L 33/105, C12J 1/00, A61K 36/00, A61K 47/40, A61K 47/69, A61P 31/00, A61P 31/04, A61P 39/00

(54) **COMPOSITION OF VINEGAR POWDER ENRICHED WITH BIOACTIVE COMPOUNDS ENCAPSULATED IN ALPHA-CYCLODEXTRINS, PREPARATION METHOD AND APPLICATIONS THEREOF**

(71) Applicant: J.R. Sabater, S.A.U., 30110 Cabezo de Torres (Murcia) (ES)
(72) Inventor: LOPEZ GOMEZ, Antonio, 30202 Cartagena (Murcia) (ES); MARTINEZ HERNANDEZ, Ginés Benito, 30202 Cartagena (Murcia) (ES); MARTINEZ SANCHEZ, Maria de los Angeles, 30202 Cartagena (Murcia) (ES); LOPEZ CANOVAS, David Antonio, 30320 Alamo de Murcia (Murcia) (ES)
(74) Representative: Herrero & Asociados, S.L.

(57) **Abstract**

The present invention provides for a composition comprising vinegar powder enriched with bioactive compounds encapsulated in α-CDs. Likewise, the invention refers to the method for obtaining the composition, starting from powdered foods or agri-food by-products and using vinegar itself as a solvent in combination with α-CDs and the application of ultrasound during the extraction of the bioactive compounds. The vinegar obtained after carrying out the extraction is filtered and then spray-dried, the α-CDs acting as an encapsulation matrix for the bioactive compounds. Finally, the present invention refers to the uses of said composition in the surface decontamination of foods and in the formulation of food products.

## Description

### FIELD OF THE INVENTION

The present invention is within the field of food additive preparation technology, in particular it refers to a new composition of vinegar powder enriched with bioactive compounds encapsulated in α-cyclodextrins (α-CDs). The addition of this new composition to certain food, refrigerated or not, significantly increases their shelf life, due to the notable antioxidant and antimicrobial effect of this composition. In addition, this additive can avoid or significantly reduce the use of table salt in certain foods. The present invention also provides the method of preparation of said composition, based on the use of the liquid vinegar itself as a solvent in combination with α-CDs and the application of ultrasound during the solid-liquid extraction, as well as its different applications in the decontamination of solid foods and formulation of food products.

### BACKGROUND OF THE INVENTION

In the state of the art, different solutions have been proposed to obtain enriched vinegars from different types of fruits and from various vegetable raw materials.

Thus, patent application CA2880180A1 refers to a preparation of a vinegar powder from vinegar neutralized (or buffered) with sodium or potassium hydroxide and which is enriched with free acid derived from vinegar. The patent application US2010/0310738A1 refers to a process for obtaining buffered vinegar powder, by spray-drying, which is enriched with liquid vinegar, to increase its free acetic acid content. Patents CN 102399676A, ES 2606453T3 and EP2880146B1 also refer to a vinegar powder enriched with liquid vinegar.

Similarly, patent application WO 2021/262144A1 refers to a vinegar powder enriched with ascorbate, glycosylated anthocyanidins, carotenes, and phenolic compounds, to be specifically used as a preservative for meat products. In this case, the enrichment of the vinegar with the aforementioned compounds is obtained simply by mixing the liquid vinegar with dry vegetable sources of these compounds, such as, for example, red berries and grapes, etc., with moisture contents not exceeding 15%, as sources of anthocyanidins; tomatoes, among other plant sources, as a source of carotenoids; and grapes, apples, tea, among other plant sources, as sources of complex phenolic compounds; and lemon or orange, among other plant sources, as sources of ascorbate. Patent ES 2657853T3 also refers to an enriched vinegar powder that is obtained simply by mixing it with lemon juice powder.

However, the proposals existing in the state of the art for vinegar compositions enriched with bioactive compounds have relatively limited antioxidant and antimicrobial activity. This is mainly due to the fact that the bioactive compounds from vegetable raw materials have a low solubility in liquid vinegar, and, therefore, they become insolubilized and do not remain in the composition of the final product.

In the scientific literature it has been revealed that ultrasound-assisted extraction (UAE) is a very interesting and environmentally friendly method to obtain bioactive compounds from agro-industrial by-products on an industrial scale *(*Maraulo et al (2021) "β-cyclodextrin enhanced ultrasound - assisted extraction as a green method to recover olive pomace bioactive compounds", Journal of Food Processing and Preservation, 45(3), e15194; https://doi.org/10.1111/jfpp.15194). UAE makes it possible to reduce the extraction times and increase the yield of said extraction. This technique, due to the acoustic cavitation phenomenon that it causes in plant cell walls, improves the permeability of bioactive compounds and the access of solvents, increasing the mass transfer and the extraction of these compounds.

On the other hand, the literature has also shown that cyclodextrins (CDs) can be used together with water as an extraction solvent to promote the extraction of both hydrophilic and hydrophobic compounds in a single step *(*Mourtzinos, I., Anastasopoulou, E., Petrou, A., Grigorakis, S., Makris, D., & Biliaderis, C. G. (2016). Optimization of a green extraction method for the recovery of polyphenols from olive leaf using cyclodextrins and glycerin as co-solvents. Journal of Food Science and Technology, 53(11), 3939-3947. https://doi.org/10.1007/s13197-016-2381-y*).* But all the works in the literature refer to the use of β-cyclodextrins (β-CDs) as an extraction adjuvant in aqueous solution. Indeed, the literature has shown that, compared to the use of organic solvents, extraction with an aqueous solution of β-CDs is more sustainable and safer (Diamanti, A. C., Igoumenidis, P. E., Mourtzinos, I., Yannakopoulou, K., & Karathanos, V. T. (2017). Green extraction of polyphenols from whole pomegranate fruit using cyclodextrins. Food Chemistry, 214, 61-66. https://doi.org/10.1016/j.foodchem.2016.07.0)*.*

Patent WO0164043A1 proposes a process for preparing a persimmon vinegar powder enriched with persimmon leaf extract, which simply consists of adding a persimmon leaf extract to concentrated persimmon vinegar and then carrying out spray drying. But the solubility of the phenolic compounds (in this case the bioactive compounds with healthy effects on the health of the consumer of this type of vinegar) is limited since it is simply a mixture of the extract with the concentrated vinegar, and does not perform solid-liquid extraction of bioactive compounds (neither from persimmon nor persimmon leaves). This patent WO 0164043A1 proposes the use of β-CDs (which it calls 7-cyclodextrins, indicating that the cyclodextrin it recommends using has seven glucose molecules), to trap aromatic molecules from persimmon leaves and avoid unpleasant taste sensations in the final persimmon vinegar being obtained.

Based on the foregoing, and the needs existing in the state of the art, the authors of the present invention have developed a new composition of vinegar powder enriched with bioactive compounds encapsulated in α-cyclodextrins (α-CDs). This innovative approach is based on the UAE of bioactive compounds from foods and agro-industrial by-products, using liquid vinegar itself as a solvent together with α-CDs. UAE improves extraction by increasing the permeability of bioactive compounds, while α-CDs act as coadjuvants for their solubilization. Furthermore, the vinegar obtained after carrying out the extraction of bioactive compounds is filtered and spray-dried, with the α-CDs acting as an encapsulation matrix for said compounds.

Until now, vinegar had not been used as a solvent with the UAE technique and, at the same time, α-CDs as coadjuvants in the solid-liquid extraction process, to achieve a better extraction of bioactive compounds. Therefore, in the cited state of the art, the extraction of these bioactive compounds is very limited, and the antioxidant and antimicrobial activity of enriched vinegar powder is also lower than that obtained with the process of obtaining vinegar of the present invention. Furthermore, since they are not encapsulated in cyclodextrins, these bioactive compounds have lower stability and, therefore, lower concentration and antimicrobial and antioxidant activity in the food to which they are incorporated.

The composition of the present invention has a surprising antioxidant and antimicrobial activity, which prolongs the shelf life of the foods to which it is added. Unlike other proposals of the state of the art, this composition encapsulates the bioactive compounds in α-CDs, guaranteeing their stability and increasing their concentration and activity in the final product, which gives rise to a multitude of important food applications.

### OBJECT OF THE INVENTION

The object of the invention is a composition of vinegar powder that comprises vinegar powder enriched with bioactive compounds encapsulated in α-CDs, in a proportion by weight with respect to the total dry weight of the composition of 96 to 99.2%, and water, in a proportion by weight with respect to the total dry weight of the composition of 0.8 to 4%.

The invention also includes a procedure for obtaining the composition of vinegar powder, which includes the steps of:
a) Obtaining foods or by-products from the agri-food industry being dehydrated and ground until reaching a moisture content of 3% to 12% on a wet basis and a granulometry of between 150 µm and 450 µm,
b) Obtaining vinegar of agricultural origin,
c) Completely dissolving α-CDs in the vinegar obtained in b), in a proportion of 5 to 15% (weight of α-CDs: vinegar volume), preferably 8 to 10%, at 22-25 °C, stirred at 1000 to 3500 rpm, for a time of 2 to 2.5 h,
d) Mixing the ground and dehydrated food or by-product of the agri-food industry from step a), in a proportion of 1:10 to 1:60 (weightvolume), preferably in a proportion of 1:30 to 1:50, with the vinegar that includes the dissolved α-CDs obtained in c), stirred at 3000 to 3500 rpm and at a temperature of 22 to 25 °C, for 30 to 60 s,
e) Carrying out the extraction of bioactive compounds by treating the mixture obtained in d) with ultrasound at 35 kHz, at a temperature of 30 to 35 °C, for 15 to 30 minutes,
f) Filtering the solution obtained in e) through a 50 to 150 µm filter, at 22 - 25 °C for 15 to 30 minutes,
g) Mixing the liquid extract obtained in f), after separating the solids retained in the filter, with 5-15% (weightvolume) of α-CDs, preferably 8-10%, stirred at 3000 to 4000 rpm, at 22-25 °C, for 15 to 30 minutes, until obtaining a homogeneous liquid, without the presence of lumps and
h) Carrying out microencapsulation by atomization of the liquid obtained in step g), in an spray dryer under the following conditions: drying air inlet temperature of 140 to 170 °C, drying air outlet temperature of 70-90 °C, pressure of the compressed air for atomization of 2 to 4 bar, and liquid feed rate to be sprayed of 2 to 5 L/h for every 100 m³/h of hot drying air, until obtaining a dry powder product with a particle size of 35 to 400 µm and a moisture content of 0.8 to 4%.

The invention also includes a method for the surface decontamination of solid foods, which comprises:
a. Directly applying the composition of vinegar powder of the invention to the solid food in a proportion by weight between 0.2 and 0.5%, or
b. Apply the composition of vinegar powder previously dissolved in water in a proportion by weight equal to or less than 2%.

Also the object of the invention is a food product that comprises in its formulation the composition of vinegar powder of the invention.

The use of the composition of vinegar powder of the invention for the surface decontamination of food is also an object of the invention.

Finally, the object of the invention is the use of the composition of vinegar powder of the invention in the formulation of food products.

### BRIEF DESCRIPTION OF THE FIGURES

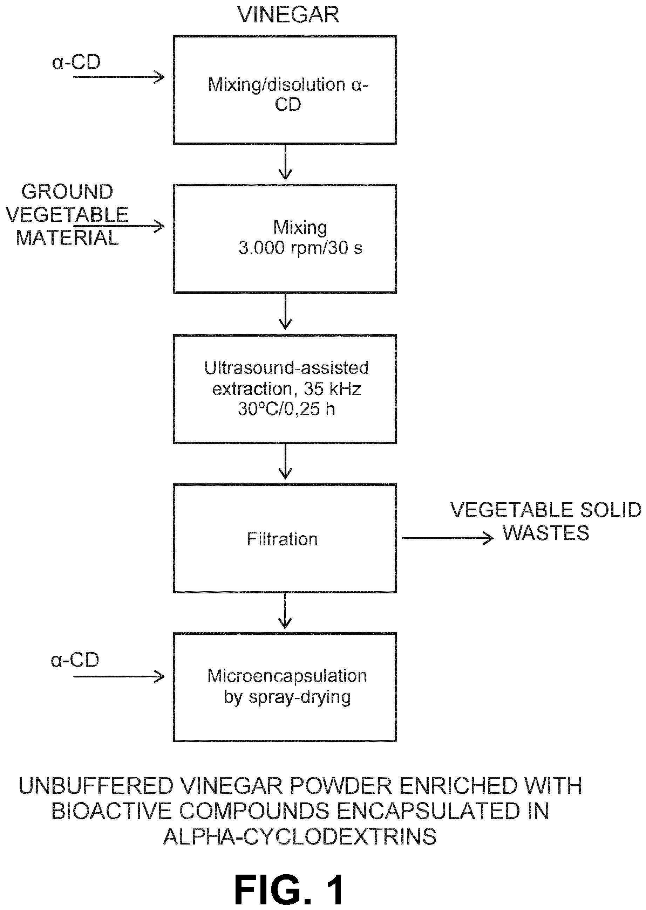

**Figure 1****.** Flow diagram of the manufacturing process of encapsulated enriched unbuffered vinegar powder proposed by this invention, using, for example, liquid vinegar and alpha-cyclodextrin (α-CD) as a coadjuvant in the process of ultrasound extraction of bioactive compounds from of ground plant material, and subsequent spray drying encapsulating the enriched vinegar in a α-CD matrix.

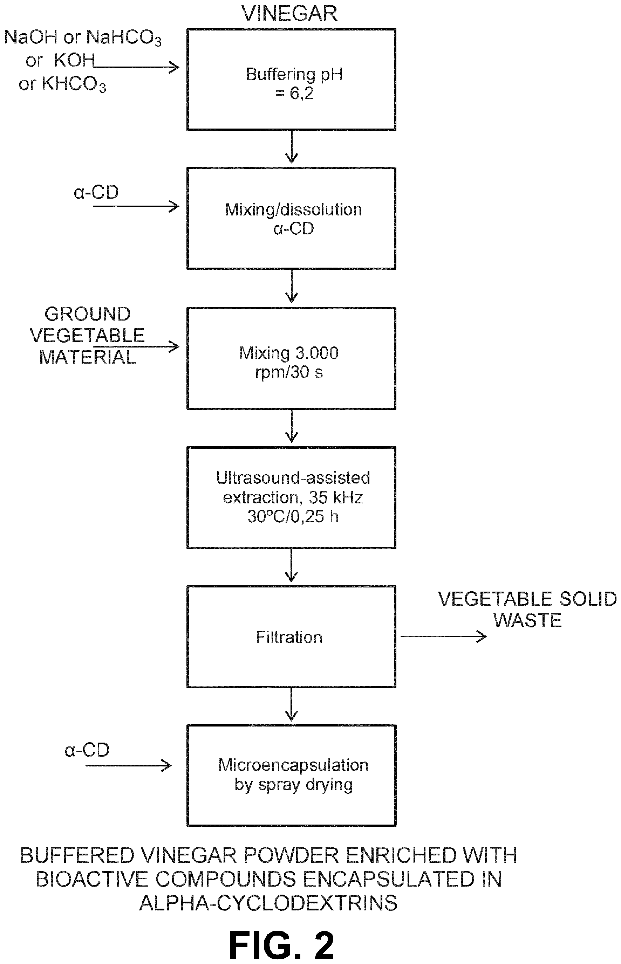

**Figure 2****.** Flow diagram of the manufacturing process of encapsulated enriched buffered vinegar powder proposed by this invention, using, for example, buffered liquid vinegar and alpha-cyclodextrin (α-CD) as a coadjuvant in the process of ultrasound extraction of bioactive compounds from of ground plant material, and subsequent spray drying encapsulating the enriched vinegar in a α-CD matrix.

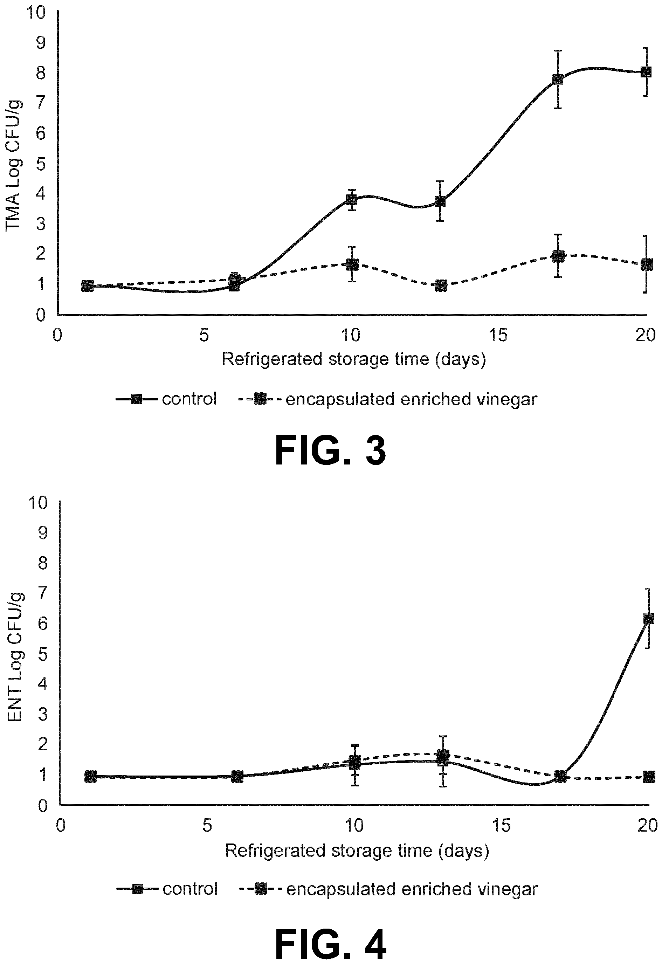

**Figure 3****.** Evolution of the microbiological count of Total Mesophilic Aerobes (TMA) (Log CFU/g) of the control béchamel croquettes formulated with NaCl ("control", solid line) and of the croquettes made with encapsulated enriched vinegar ("encapsulated enriched vinegar", dashed line) for 20 days of refrigerated storage at 4 °C. Average values (n =3) and standard deviation are shown.
**Figure 4****.** Evolution of the microbiological count of Enterobacteriaceae (ENT) (Log CFU/g) of the control béchamel croquettes formulated with NaCl ("control", solid line) and of the croquettes made with encapsulated enriched vinegar ("encapsulated enriched vinegar", dotted line) for 20 days of refrigerated storage at 4 °C. Average values (n =3) and standard deviation are shown.

### DESCRIPTION OF THE INVENTION

To overcome the limitations found in the state of the art in relation to the extraction of bioactive compounds and their stability and availability in vinegar compositions, the present invention proposes a new composition of vinegar powder enriched with bioactive compounds encapsulated in α-CDs, to be applied in surface decontamination of foods and agri-food by-products, as well as in the formulation of different types of foods, which will also make it possible to use less table salt in its composition, and have a longer shelf life than that obtained with traditional recipes.

Thus, in a first aspect of the invention a composition of powder vinegar is provided for (hereinafter, composition of the invention) comprising:
- Vinegar powder enriched with bioactive compounds encapsulated in α-CDs, in a proportion by weight, with respect to the total dry weight of the composition, of 96 to 99.2%, and
- Water, in a proportion by weight, with respect to the total dry weight of the composition, of 0.8 to 4%, water being the moisture content on a dry basis of the composition.

In this composition, the vinegar can be buffered or not, and sodium hydroxide, sodium bicarbonate, potassium hydroxide or potassium bicarbonate can be used to buffer the vinegar. Furthermore, vinegar can be made from alcohol, cider, wine, or any product or drink of agricultural origin resulting from double alcoholic and acetic fermentation.

The α-CDs used in this composition are characterized by having 6 glucose units; a molecular weight of 972; a number of water molecules in the cavity of 6; a solubility in water at 25 °C of 145 g/L; a diameter of the central cavity (nm) of 0.5 - 0.6; an outer diameter (nm) of 1.4 - 1.5; and a height of the toroidal shape (nm) of 0.8.

If the physical-chemical characteristics of α-CDs and β-CDs (used in the state of the art) are compared, it can be seen that β-CDs have a solubility in water of only 18.0 g/L (at 25°C) (according to the specification sheet of the manufacturer and supplier of this beta-cyclodextrin, Roquette Frères, France), while α-CDs have a water solubility of 145 g/l (at 25 °C) (according to the manufacturer and supplier Wacker Chemie AG, Germany). Probably due to this greater water solubility of α-CD (approximately 10 times greater than that of β-CD), the composition of the invention shows that, surprisingly, the bioactive compounds encapsulated in α-CD are more soluble in water and, therefore, they reach more easily, in aqueous medium, oxidative enzymes and the cell membranes of spoilage and pathogenic microorganisms, normally present in the aqueous fraction of foods. In this way, the authors of the present invention have demonstrated that the same bioactive compounds, when encapsulated in α-CDs, exert a surprisingly greater antioxidant and antimicrobial effect than when encapsulated in β-CDs, using equal doses (or concentrations) of bioactive compounds in aqueous medium. This is one of the great differences (and advantages) that the composition object of this invention has, when compared to other proposals where the encapsulating agent is β-CD. Furthermore, the cost per kilogram of α-CD has almost equaled in recent years the cost of the initially cheaper β-CD. Therefore, the composition of the present invention (which includes α-CD as an encapsulating agent and coadjuvant for the extraction of bioactive compounds) is truly competitive, which facilitates its application at the food industry level, since it requires, in this case, certain lower doses (this implies lower costs) than if it included β-CD. In fact, there is no encapsulated vinegar composition enriched with bioactive compounds that includes β-CD on the market, which demonstrates its lack of industrial and commercial viability.

The bioactive compounds included in the composition of vinegar powder of the invention are essential and non-essential biological compounds that are found in nature, are part of the food chain and have a positive effect on human health, preferably selected from phenolic compounds, alkaloids, terpenes, and terpenoids, aromatic and aliphatic compounds, nitrogen-containing compounds (including peptides and polypeptides), organosulfur compounds, fatty acids, or a combination of two or more thereof.

Indeed, for example, terpenoids have recognized antioxidant activity, such as tocotrienol, tocopherols, carotenoids, limonoids and phytosterols. The compounds α-carotene, β-carotene, β-cryptoxanthin, and lutein also belong to the group of carotenoids. In general, bioactive compounds also have a certain antimicrobial activity recognized by scientific literature.

These bioactive compounds can come from plants, fungi, algae, fish, meat, dairy products, or foods in general, as well as byproducts obtained from the agri-food industry. They can come from agroindustrial by-products, such as, for example, by-products from the extraction of pomegranate juices, by-products from the marketing of fresh mushrooms, by-products from winemaking, by-products from the canned vegetable industry (such as, for example, tomato, artichoke, pepper, pear, apple), by-products from the citrus juice industry (orange, tangerine, lemon, grapefruit), by-products from the fresh fruit and vegetable industry (such as, for example, citrus, watermelon, melon, tomato, carrot, broccoli, mushroom, tropical fruits, avocados, and leaf vegetables, such as lettuce).

Therefore, based on the foregoing, the composition of the present invention only includes ingredients for food use, such as vinegar powder of agricultural origin, buffered or not, α-CDs (approved as a new agri-food ingredient that increases the content of dietary fiber in the foods in which it is incorporated, according to the Opinion of the European Food Safety Agency: *"Opinion of the Scientific Panel on Dietetic Products, Nutrition and Allergies related to the safety of alpha-cyclodextrin, EFSA Journal, https:*//*doi.org*/*10.2903*/*j.efsa.2007.537*")*,* and bioactive compounds encapsulated in α-CDs, which have been extracted from foods in general or from by-products of the agri-food industry. Therefore, the composition of the present invention does not have legal limits for use and ingestion, as occurs, for example, with compositions that include β-CDs.

A second aspect of this invention refers to the method for obtaining the composition that is the subject of this patent, which is characterized by:
a. obtaining foods in general or by-products from agri-food industry being dehydrated, up to a moisture content of 3% to 12% on a wet basis (the moisture content on a wet basis in percentage refers to the kg of water contained in 100 kg of the wet product), and ground, until reaching a granulometry where more than 95% of the weight passes through a 45 mesh (354 µm) sieve (granulometry between 150 µm and 450 µm),
b. obtaining vinegar of agricultural origin, preferably with 6 to 20% of acetic acid, more preferably alcohol vinegar with 9 to 12% of acetic acid,
c. completely dissolving α-CDs in the vinegar obtained in b), in a proportion of 5 to 15% (weightvolume), preferably 8 to 10%, at 22-25°C, stirred at 1000 to 3000 rpm, for a period of time from 2 to 2.5 hours,
d. Mixing the powdered agroindustrial food or by-product (ground and dehydrated) (for example, from pomegranate or fresh mushroom), as described in step a) above, in a proportion of 1:10 to 1:60 (weightvolume), preferably in a proportion of 1:30 to 1:50, with the vinegar that includes the dissolved α-CDs as described in c), stirred at 3000 to 3500 rpm and at a temperature of 22 to 25°C, for 30 to 60s,
e. Carrying out the extraction of bioactive compounds by treating the mixture obtained in d) with ultrasound at 35 kHz, at a temperature of 30 to 35 °C, for 15 to 30 minutes.
f. Filtering the solution obtained in e) through a 50 to 150 µm filter, at 22 - 25 °C for 15 to 30 minutes,
g. Mixing the liquid extract obtained in f), after separating the solids retained in the filter, with 5-15% (weightvolume) of α-CDs, preferably 8-10%, stirred at 3000 to 4000 rpm at 22 at 25 °C, for 15 to 30 minutes, until obtaining a homogeneous liquid, without the presence of lumps and
h. Carrying out microencapsulation by atomization (spray-drying) of the liquid obtained in step g) above, in a spray dryer, for example in a two-fluid nozzle spray dryer in co-current mode, or any other type of spray dryer, under the following conditions: drying air inlet temperature of 140 to 170 °C, drying air outlet temperature of 70-90 °C, pressure of the atomization compressed air of 2 to 4 bar, and liquid feed rate to be atomized of 2 at 5 Uh, preferably 3 to 4 Uh per 100 m³/h of hot drying air until obtaining a dry powder product of vinegar r enriched with bioactive compounds encapsulated in α-CDs with a particle size of 35 to 400 µm, and a moisture content of 0.8 to 4%.

In a particular embodiment of the method of the invention, step a) can start from dried red pepper or paprika, or black pepper, or nutmeg, or ginger, turmeric, or celery, or any other vegetable from which oleoresins are extracted, to obtain a vinegar powder enriched with coloring matter and bioactive compounds of the oleoresin from paprika, from black pepper, from nutmeg, from ginger, from turmeric, or from celery, or from any other vegetable, respectively, encapsulated in α -CDs. Thus, with this method, water-soluble liquid oleoresins are obtained before carrying out the spray drying.

When the composition of the invention is added in powder form or in aqueous solution to solid foods (fresh meats and fish, meat products, dairy products, fresh fruits and vegetables, etc.), it has a high antioxidant and antimicrobial activity that lasts during the storage time of the corresponding food.

Thus, a third aspect of the present invention refers to the method for the surface decontamination of solid foods, which comprises:
a. Directly applying the composition of vinegar powder of the invention to the solid food, in a proportion by weight between 0.2 and 0.5%, or
b. Applying an aqueous solution of the composition of vinegar powder of the invention, previously completely dissolved by stirring in water in a proportion by weight equal to or less than 2% (2 g/100 g of water or 20 g/kg of water).

In a particular embodiment, in case b), where the composition of the invention is applied in the form of a solution, the method comprises the following steps:
i. Bathing solid foods in the aqueous solution for a time of 0.5 to 1.5 minutes, preferably 0.8 to 1.2 minutes, or, alternatively, applying the aqueous solution prepared by showering, at a rate of 1-1.2 liters of solution per 1000 kg of food, and
ii. drying and cooling the foods treated with the aqueous solution in i), for example by means of the well-known method called vacuum cooling, managing to dry and cool at the same time, or, alternatively, drying the foods treated with the aqueous dissolution using hot air at a temperature between 25 and 55 °C.

In a surprising and novel way, the composition of the present invention, once applied to solid foods with any of the previous methods, by bath or shower, achieves a high initial reduction (through the antimicrobial effect of the composition) of the surface microbial load of more than 2 logarithmic units (log CFU/cm² or log CFU/g of product; where CFU means Colony Forming Units), in treated foods.

Furthermore, with the composition of the present invention, the decontamination effect is prolonged over time, after the initial intense microbial decontamination bath, through the consequent controlled release of the bioactive compounds that have been encapsulated within the α-CDs, achieving control microbial growth on the surface of foods preserved in refrigeration. The composition of vinegar powder enriched with bioactive compounds encapsulated in α-CDs, object of this invention, better preserves food due to its antioxidant activity and its antimicrobial activity on total aerobic microorganisms, lactic acid bacteria, enterobacteria, psychrophiles, molds and yeasts, and also on pathogenic microorganisms, such as *Salmonella* sp., *Escherichia coli* and *Listeria monocytogenes.*

In another aspect of the invention, the use of the composition of vinegar powder enriched with bioactive compounds encapsulated in α-CDs of the invention is provided for the formulation of meat products, foods in general, or prepared dishes, such as croquettes, to increase their shelf life as products refrigerated and preserved at 4 °C, since this composition preserves the product better, due to its antioxidant activity and antimicrobial activity on total aerobic microorganisms, lactic acid bacteria, enterobacteria, psychrophiles, molds and yeasts, and also on pathogenic microorganisms, such as *Salmonella sp., Escherichia coli* and *Listeria monocytogenes.* Thus, for example, in a particular embodiment, the composition of vinegar powder enriched, for example, with bioactive pomegranate compounds encapsulated in α-CDs, can be mixed with the other ingredients of the food to be prepared, in a variable proportion by weight depending on the food in question.

In a particular aspect of this invention, the common salt (sodium chloride) of the traditional food recipe, for example, croquettes and other breaded foods, vegetable salads, and other foods in general, can be replaced by the same or different proportion by weight of vinegar powder enriched with bioactive compounds of mushroom encapsulated in α-CDs, giving the food, thus formulated, sensory characteristics similar to those provided by common salt, but without adding sodium, with the consequent benefits for the health of the consumer. Furthermore, in a surprising and novel way, the composition proposed by this invention, once applied to the formulation of these foods (e.g. croquettes), manages to significantly increase their shelf life as a refrigerated product, preserved at 4 °C, increasing its shelf life by more than a week, compared to the normal shelf life of refrigerated croquettes, also improving its food safety.

Finally, based on the foregoing, another main aspect of the invention provides for a food product that comprises in its formulation the composition of vinegar powder of the invention. In a particular embodiment, the table salt of the original formulation of said food product is replaced with the composition of vinegar powder. In a preferred embodiment, the bioactive compounds that provide said salty touch are obtained from mushrooms or byproducts of the mushroom industry.

### EXAMPLES

Examples are listed below, as non-exclusive ways of carrying out and applying the invention.

### Preparation of the solution of alcohol vinegar (buffered or unbuffered) with α-CDs

First, an alcohol vinegar (hereinafter unbuffered) with an acetic acid content of 10% is prepared by diluting alcohol vinegar with a high acetic acid content (≈20%) with deionized water in a 1:1 ratio (volume/volume). To prepare buffered alcohol vinegar, a 1 Molar solution of potassium bicarbonate (KHCO₃) is added to the alcohol vinegar, such as the one previously prepared with 10% of acetic acid, to increase the pH up to 6.2 while controlling and maintaining the temperature of the vinegar solution during this neutralization process at 22 °C, thus obtaining the buffered vinegar.

The preparation of the α-CD solution in unbuffered or buffered alcohol vinegar is carried out at room temperature, adding and dissolving α-CD in a proportion of 10% (weight:volume) to any of the alcohol vinegars prepared above (unbuffered or buffered), as indicated in the previous paragraph.

### Example 1: Obtaining unbuffered alcohol vinegar powder, enriched with bioactive compounds from pomegranate or mushroom by-products encapsulated in α-CDs

The following steps are carried out, as detailed in Figure 1.
1. Preparing an unbuffered alcohol vinegar solution (with 10% acetic acid content) with 10% (weight/volume) of α-CD as a coadjuvant, for the extraction of bioactive compounds. It is carried out by stirring at 1000 rpm in an MS-H280-Pro magnetic stirrer, at room temperature (23 °C) until complete dissolution is achieved.
2. Mixing the solution prepared in (1) of unbuffered alcohol vinegar and α-CD with ground dehydrated pomegranate or mushroom by-products (granulometry with more than 95% of the weight passing through a 45 mesh sieve), at a 1:50 ratio (ground plant material:vinegar), at room temperature, and stirring at 3000 rpm for 30 s in a T 25 ULTRA-TURRAX^{®} stirrer.
3. Ultrasound-assisted extraction of bioactive compounds, applying ultrasound to the mixture of unbuffered vinegar with α-CD with dried and ground by-products of pomegranate or mushroom obtained in (2), in a Bandelin Sonorex Digiplus DL 514 BH ultrasound equipment with a frequency of 35 kHz for 0.25 h and at a temperature of 30 °C and 100% power.
4. Filtration of the unbuffered vinegar solution obtained in (3), at room temperature, and using a 0.100 mm pore size sieve.
5. Spray drying of this solution, obtained in (4), of unbuffered and filtered enriched liquid vinegar, using α-CD as encapsulation matrix in a proportion of 10% (weight/volume), in a two-fluid nozzle pilot spray dryer in co-current mode, under the following conditions: drying air inlet temperature of 150 °C, drying air outlet temperature of 80 °C, pressure of the atomization compressed air of 4 bar, and liquid feed rate to be atomized of 3 Uh, for every 100 m³/h of hot drying air. A dry powder product is obtained, with a powder particle size after spray drying d(0.9) of 45 to 400 µm in the vinegar enriched with bioactive compounds encapsulated in α-CD, and a moisture content of 0.8 to 4% (on a wet basis).

### Example 2: Obtaining buffered alcohol vinegar powder, enriched with bioactive compounds from pomegranate or mushroom by-products encapsulated in α-CD.

The following steps are carried out, as detailed in Figure 2:
1. Preparing a buffered alcohol vinegar solution (with a pH of 6.2) with 10% (weight/volume) of α-CD as an adjuvant, for the extraction of bioactive compounds. It is carried out by stirring at 1,000 rpm in an MS-H280-Pro magnetic stirrer, at room temperature (23 °C), until complete dissolution is achieved.
2. Mixing the solution prepared in (1) of buffered alcohol vinegar and α-CD with ground dehydrated pomegranate or mushroom by-products (granulometry with more than 95% of the weight passing through a 45 mesh sieve), at a ratio 1:50 (ground plant material: vinegar), at room temperature (23 °C), and stirring at 3000 rpm for 30 s in a T 25 ULTRA-TURRAX^{®} stirrer.
3. Ultrasound-assisted extraction of bioactive compounds, applying ultrasound to the mixture of buffered vinegar with α-CD with dried and ground by-products of pomegranate or mushroom obtained in (2), in a Bandelin Sonorex Digiplus DL 514 BH ultrasound equipment with a frequency of 35 kHz for 0.25 h and at a temperature of 30 °C and 100% power.
4. Filtration of the buffered vinegar obtained in (3) at room temperature (23 °C) using a 0.100 mm pore size sieve.
5. Spray drying this solution, obtained in (4), of buffered and filtered enriched liquid vinegar, using α-CD as encapsulation matrix in a proportion of 10% (weight/volume), in a pilot plant spray dryer with two-fluid nozzle in co-current mode, under the following conditions: drying air inlet temperature of 150 °C, drying air outlet temperature of 80 °C, pressure of the atomization compressed air of 4 bar, and liquid feed rate to be atomized of 3 L/h, for every 100 m³/h of hot drying air. A dry powder product is obtained, with a powder particle size after atomization d(0.9) of 45 to 400 µm in the vinegar enriched with bioactive compounds encapsulated in α-CD, and a moisture content of 0.8 to 4% (on a wet basis).

### Example 3: Application in powder form of alcohol vinegar, unbuffered or buffered, enriched with bioactive compounds encapsulated in α-CD to extend the shelf life of packaged fresh cut lettuce.

The following steps are carried out:
1. Starting from alcohol vinegar powder, unbuffered or buffered, enriched with bioactive compounds of pomegranate or mushroom encapsulated in α-CD, obtained as described in the previous examples 1 and 2.
2. Starting from fresh cut lettuce into uniform strips that have been washed by immersing them in drinking water at 4 °C for a period of 30 seconds. Afterwards, this cut lettuce is centrifuged manually, at room temperature, until the surface moisture is completely eliminated.
3. The cut lettuce described in (2) is packaged in 750 mL heat-sealable rPET containers. Then, the enriched vinegar powder - unbuffered or buffered - described in (1) is added directly to this packaged cut lettuce in a proportion of 0.2% (0.2 g/100g of packaged product or 2 g/kg of packaged product), or 0.4% (0.4 g/100 g of packaged product or 4 g/kg of packaged product), or 1% (1 g/100 g of packaged product or 10 g/kg of packaged product). The fresh cut lettuce described in (2) packaged in this type of containers, without the addition of enriched vinegar powder that is the object of this invention, is considered as a control treatment.

Next, the aforementioned containers with cut lettuce were heat-sealed in a heat-sealer with a 39 µm thick plastic film, and they were stored in a cold room at 4 °C for 5 days. On the initial day (day 0) of packaging and after 5 days of refrigerated storage, the determination of the surface microbiology of the cut lettuce (analysis of total mesophilic aerobic microorganisms, TMA; psychrophiles, PSI; enterobacteria, ENT; and molds) and the sensory analysis thereof were performed.

The analysis of the counts of microorganisms present on the surface of the packaged and refrigerated cut lettuce was performed by taking 25 g of cut lettuce and immersing them in 225 mL of peptone water at 4 °C for 1 hour, and with stirring; next, the corresponding serial dilutions in peptone water and inoculation in the appropriate growth media for each microorganism were carried out. Determination of TMA and PSI was carried out plating in Plate Count Agar (PCA) culture medium, and it was incubated at 30 °C for 48 h for TMA, and at 4 °C and for 7 days for PSI. ENT counts were assessed by plating on Violet Red Bile Dextrose (VRBD) medium, and incubating at 37°C for 48 h. The molds were sown on the surface in the rose bengal medium, and then incubated at 25 °C for 7 days. The results of the microbiological analyzes were expressed as the logarithm of colony-forming units per gram (Log CFU/g).

The direct addition of vinegar powder enriched with bioactive compounds encapsulated in α-CD, to the cut lettuce had an immediate antimicrobial effect. The control lettuce, without encapsulated enriched vinegar, had a load of more than 5 Log CFU/g of TMA microorganisms on day 0 of packaging, compared to the lettuce treated with enriched vinegar powder, which showed counts of 3.08-3.89 Log CFU /g. After 5 days of refrigerated storage, packaged lettuce with moderate doses of enriched vinegar (0.4% or 0.4 g/100 g of packaged product or 4 g/kg of packaged product) and high doses (1% or 1 g/100 g of packaged product or 10 g/kg of packaged product) had an TMA count of approximately 5 Log CFU/g, while cut lettuce that did not incorporate enriched vinegar powder reached a count of approximately 7 Log CFU/g. A similar effect to the TMA count was observed on the counts of psychrophilic microorganisms and enterobacteria. Furthermore, mold counts were found to remain below 1 Log CFU/g throughout the storage. That is, the application of enriched vinegar powder has the ability to limit the growth of microorganisms that compromise the quality and shelf life of packaged fresh cut salad. This example shows that, surprisingly, the application of vinegar powder enriched with bioactive compounds encapsulated in α-CD achieves a 99% reduction in the count of TMA, PSI, ENT and molds on the surface of fresh cut lettuce, extending its shelf life under refrigeration by more than 40%, compared to the control cut lettuce without addition of the enriched vinegar powder object of this invention.

Furthermore, it was observed that the application in powder form of the enriched vinegar object of this invention achieved a surprising antioxidant effect during the refrigerated storage of cut lettuce. The treatments with enriched buffered vinegar showed a low level of oxidations on the surface of the cut lettuce after 5 days of storage under the conditions described, unlike the control packaged lettuce without the addition of enriched vinegar, which showed the presence of oxidations after 3 days of storage. The application of moderate doses (0.4% or 0.4 g/100 g of packaged product) of enriched vinegar object of this invention managed to maintain an acceptable appearance of the cut and packaged lettuce, demonstrating good storage for 5 days at 4 °C.

### Example 4: Application in the form of a solution of unbuffered or buffered alcohol vinegar enriched with bioactive compounds encapsulated in α-CD, to extend the shelf life of packaged fresh cut lettuce

The following steps are carried out:
1. Starting from alcohol vinegar powder, unbuffered or buffered, enriched with bioactive compounds of pomegranate or mushroom encapsulated in α-CD, obtained as described in the previous examples 1 and 2.
2. Dissolving the composition previously described in (1) in drinking water, in a proportion of 0.1% (0.1 g/100 g of water or 1 g/kg of water), 0.2% (0.2 g/100 g of water or 2 g/kg water), 0.4% (0.4 g/100 g of water or 4 g/kg of water), 0.6% (0.6 g/100 g water or 6 g/kg of water) and 1% (1 g/100 g of water or 10 g/kg of water) (weight/volume), stirring until complete solubilization.
3. Immersing or bathing fresh cut lettuce (prepared as indicated in Example 3 above) in the aqueous solutions prepared as described in (2) for a total time of 1 minute and at 4 °C. The bath of fresh cut lettuce in drinking water, at the same temperature and for the same time, without the composition that is the object of this patent, is considered as control treatment.
4. Manually centrifugating at room temperature the fresh lettuce treated using the solutions described above, until the surface moisture in the tissue is completely eliminated.

Next, the cut and treated lettuce was packaged in 750 mL rPET plastic containers, sealed with a manual heat sealer with a 39 µm thick film, and stored at 4 °C for 5 days. On the initial day (day 0) of packaging and after 5 days of refrigerated storage, the determination of the surface microbiology of the cut lettuce was performed (analysis of total mesophilic aerobic microorganisms, TMA; psychrophiles, PSI; enterobacteria, ENT; and molds) and the sensory analysis thereof. This microbiological analysis was carried out following the same methodology described in Example 3 above. The results were expressed as Log CFU/g of fresh cut lettuce.

In this surface decontamination test by bathing in a solution of unbuffered or buffered vinegar enriched with bioactive compounds encapsulated in α-CD, an important reduction in the surface microbial load of fresh cut lettuce was observed. The treatments in which moderate proportions of this enriched vinegar of 0.4% (0.4 g/100 g of water or 4 g/kg of water) or 0.6% (0.6 g/100 g of water or 6 g/kg of water) were used achieved an important antimicrobial effect, with decreases greater than 2 Log CFU/g compared to control lettuce not treated with a vinegar solution enriched with bioactive compounds encapsulated in α-CD. On day 0 of storage, significant differences have already been observed between the control treatment and treatments with enriched vinegar, the object of this invention, since the TMA count was reduced between 1 and 2 Log CFU/g compared to the control. After 5 days of storage, the treatments with the unbuffered or buffered enriched vinegar solutions had TMA counts in the order of 4 Log CFU/g, while the control lettuce reached around 6 Log CFU/g. The surface load of PSI and ENT microorganisms was also reduced by the aforementioned decontamination bath with enriched vinegar solutions, there being differences greater than 2 Log CFU/g with respect to the counts of the control lettuce. Mold counts were less than 1 Log CFU/g until the end of storage. This example of bathing in encapsulated enriched vinegar solutions, object of this invention, is surprisingly effective in reducing up to 4 Log CFU/g of TMA and manages to extend the shelf life of cut lettuce by more than 5 days under refrigerated storage at 4 °C.

The fresh cut lettuce with the decontamination bath treatments in solutions of 0.2-0.4% of encapsulated enriched vinegar, object of this invention, maintained an acceptable appearance and free of oxidation during refrigerated storage. However, the control treatment and decontamination baths carried out with very low concentrations of 0.1%, or very high concentrations of 0.6-1%, led to the appearance of oxidations after 5 days of storage.

This decontamination bath of fresh cut lettuce, in a solution preferably with a concentration of 0.4% of encapsulated enriched vinegar, unbuffered or buffered, is effective in extending the shelf life of this fresh product, due to its antimicrobial and also antioxidant effect.

### Example 5: Application in the form of a decontamination solution of unbuffered or buffered alcohol vinegar enriched with bioactive compounds encapsulated in α-CD, to extend the shelf life of sliced mushroom

The following steps are carried out:
1. Starting from alcohol vinegar powder, unbuffered or buffered, enriched with bioactive compounds of pomegranate or mushroom encapsulated in α-CD, obtained as described in the previous examples 1 and 2.
2. Dissolving the composition previously described in (1) in drinking water, in a proportion of 0.1% (0.1 g/100 g water or 1 g/kg of water), 0.2% (0.2 g/100 g of water or 2 g /kg of water), 0.4% (0.4 g/100 g of water or 4 g/kg of water), 0.6% (0.6 g/100 g of water or 6 g/kg of water) and 1% (1 g/ 100g of water or 10 g/kg of water), stirring until its complete solubilization.
3. Immersion or bath of whole mushroom in the aqueous solutions made in (2) for a total time of 1 minute. Bathing whole mushrooms in drinking water is considered as control treatment.
4. Manual centrifugation at room temperature of the whole mushroom treated with the solutions described until the surface moisture in the mushroom is completely eliminated.
5. Cut the whole treated and dried mushroom into uniform 3 mm thick slices, using a previously disinfected kitchen knife.

Next, the mushroom slices were packaged in 750 mL rPET plastic containers, sealed with a manual heat sealer with a 39 µm thick film, and stored at 4 °C for 5 days. On the initial day (day 0) of packaging and after 5 days of refrigerated storage, the determination of the surface microbiology of the mushroom slices (analysis of total mesophilic aerobic microorganisms, TMA; psychrophiles, PSI; enterobacteria, ENT; and molds) and their sensory analysis were performed. This microbiological analysis was carried out following the same methodology described in Example 3 above. The results were expressed as Log CFU/g of mushroom cut into slices.

On day 0 of packaging, no significant differences were found in the microbial counts of TMA between the control and bath treatments with solutions of different concentrations of encapsulated enriched vinegar. But after 2 days of storage, surprisingly, it was observed that the decontamination treatment carried out with some of the solutions with vinegar enriched with encapsulated bioactive compounds from the mushroom achieved a reduction of the order of 2 Log CFU/g compared to the control treatment. After 5 days of storage, TMA counts in the control sliced mushroom reached 7 Log CFU/g, while the sliced mushroom treated with vinegar solutions enriched with bioactive compounds (mushroom) encapsulated in α-CD, had a microbiological load of 1 to 2 Log CFU/g lower, revealing a clear antimicrobial effect of the composition object of this invention.

The sensory analysis of the stored sliced mushroom also revealed surprising differences between the control samples and those that underwent treatments with encapsulated enriched vinegar solution, the object of this invention. On day 0, the control sliced mushroom samples and the mushroom treated with low-moderate proportions (from 0.1 to 0.4%) of encapsulated enriched vinegar in solution showed an adequate appearance at the time of packaging, while the treatments with solutions to the 0.6 and 1% of encapsulated enriched vinegar showed slight browning. After 5 days of storage, the control samples and the treatments with a 0.1% solution of enriched vinegar showed considerable oxidations. However, the sliced mushroom samples from the treatment with 0.2% solution were found to be free of oxidations. This antioxidant effect of the solution with the composition object of this invention was especially surprising in the sliced mushroom samples that had been treated with the encapsulated enriched buffered vinegar compositions object of this invention.

### Example 6: Application in powder form of unbuffered or buffered encapsulated enriched alcohol vinegar, object of this invention, to replace the addition of sodium chloride, or common salt, in breaded products

The following steps are carried out:
1. Starting from unbuffered or buffered alcohol vinegar powder enriched with bioactive compounds of pomegranate or mushroom encapsulated in α-CD, obtained as described in the previous examples 1 and 2.
2. The béchamel croquette dough is made by preparing two types of dough, using a kitchen robot (Thermomix^{®} TM31 Vorwerk), and following the recipe described in the patent by López-Gómez et al (2014) entitled "Composition and method for the preparation of breaded foods with low oil absorption during frying" (ES2440092B2). Control croquettes are prepared by adding NaCl (common salt) to the dough that will be the core of the croquette (hereinafter, referred to in the text as "control croquettes"). In the croquettes made with vinegar enriched with bioactive compounds encapsulated in α-CD (from unbuffered or buffered alcohol vinegar and enriched with bioactive compounds from pomegranate or mushroom), no common salt (NaCl) was added.
3. The dough is portioned and these portions are molded into croquettes and individually dipped in coating base before being breaded with wheat breadcrumbs.
4. The breaded croquettes are frozen and stored at -20 °C for 24 h, before being packaged in 750 mL rPET plastic trays, under modified atmosphere conditions (70% N2, 30% CO2), in a semi-automatic heat sealer of Efaman brand (Efabind).

The shelf life study of the packaged croquettes was carried out by storing the samples at 4 °C for 20 days. On each day of analysis (days 1, 6, 10, 13, 17 and 20), a microbiological analysis was performed (total mesophilic aerobic microorganisms, psychrophiles, enterobacteria and molds) of the control croquettes and croquettes with encapsulated enriched vinegar. For this purpose, the same methodology described in Example 3 above was used. The results were expressed as Log CFU/g of product. Figure 3 and Figure 4 show the results of the evolution of the microbial load of the croquettes, specifically, the TMA and ENT counts.

For sensory evaluation of the croquettes, they were first cooked in a hot air convection oven at 180 °C for 15 minutes.

In this example of application of enriched encapsulated vinegar powder, a surprising antimicrobial effect was observed derived from the incorporation of this composition, object of this invention, in the formulation of the core of the béchamel croquettes. Figure 3 shows that, on the first day of packaging, no significant differences were found in TMA counts (which were less than 1 Log CFU/g for the control and the encapsulated enriched vinegar treatments). However, over 20 days of refrigerated storage of croquettes, the differences found in the microbial counts of the croquettes with encapsulated enriched vinegar, compared to the control croquettes, were very significant. Starting on day 10, an increase in TMA counts was seen in the control croquettes that reached approximately 4 Log CFU/g, while the counts observed in the croquettes formulated with encapsulated enriched vinegar object of this invention remained around 1 Log CFU/g. At the end of storage, after 20 days, the TMA counts in these croquettes with encapsulated enriched vinegar were less than 3 Log CFU/g, extraordinarily lower than the counts obtained for the control croquettes formulated with common salt, which were above 8 Log CFU/g. At the end of refrigerated storage, PSI microorganism counts also reached values greater than 7 Log CFU/g in the control croquettes, while the results in the croquettes with encapsulated enriched vinegar were around 1 Log CFU/g. Also, surprising differences were observed in the ENT counts shown in Figure 4, where it can be seen that after 20 days of storage the counts in the control croquettes reached approximately 5 Log CFU/g, compared to the croquettes that were prepared with encapsulated enriched vinegar, which showed a count for these microorganisms of the order of 1 Log CFU/g. During the first 20 days of storage, molds in the croquette with enriched vinegar were outside the detection limit, reaching around 4 Log CFU/g in the control croquette with common salt on day 20 of storage.

The sensory analysis did not reveal significant differences in the appearance of the control croquettes or those that incorporated enriched encapsulated vinegar powder in their formulation, although differences were observed regarding the creaminess and flavor of the dough during and after chewing. When comparing the control croquettes and the croquettes with vinegar enriched with bioactive compounds (encapsulated) from pomegranate and mushroom, it was found that the addition of vinegar enriched with bioactive mushroom compounds increased the creaminess and softness of the core of the croquettes. The enrichment with bioactive compounds from pomegranate or mushroom and the buffered or unbuffered state of the vinegar influenced the sensory notes that were appreciated in the croquettes. The croquettes that incorporated buffered vinegar enriched with encapsulated bioactive compounds from pomegranate were more neutral, somewhat bitter and astringent, while the samples with unbuffered vinegar enriched with pomegranate were soft and fruity, compared to the control croquettes. The croquettes made with vinegar enriched with mushrooms were tasty and had a high general acceptance, higher than the control croquettes. Surprisingly, no differences were found in the salty taste between the croquettes formulated with buffered vinegar enriched with mushroom and the control croquettes, showing that the composition object of the present invention managed to match the salty sensation of the croquettes that did incorporate common salt in the formulation. The addition of buffered vinegar enriched with mushroom gave rise to béchamel croquettes with a notable salty flavor, there being no bitter or unpleasant flavors that are usually related to the use of potassium salts that are commonly used to replace common salt (NaCl), and that also reached a high level of acceptance, surpassing the control croquettes due to the tasty notes provided by the mushroom used in the composition object of this invention, which were also creamier. This sensory evaluation test not only showed that the buffered vinegar enriched with mushroom managed to match the salty flavor sensation of the control croquettes (with common salt), but that NaCl was eliminated in the formulation of the béchamel croquettes.

These non-exclusive examples of manufacturing and application of vinegar (unbuffered or buffered) enriched with bioactive compounds encapsulated in α-CD, object of this invention, show that this new vinegar powder has an antimicrobial and antioxidant effect that makes it possible to extend the shelf life of certain refrigerated foods, avoiding the need to use chemical additives for preservation. Furthermore, some compositions of this invention confer salty sensory characteristics analogous to those achieved with the use of common salt when incorporated into some foods. This will reduce the use of common salt in some prepared food formulations, resulting in healthier foods, with a lower sodium content and sensory similarity.

## Claims

1. Composition of vinegar powder that includes:
- Vinegar powder enriched with bioactive compounds encapsulated in α-CDs, in a proportion by weight, with respect to the total dry weight of the composition, of 96 to 99.2%, and
- Water, in a proportion by weight with respect to the total dry weight of the composition, from 0.8 to 4%.

2. Composition of vinegar powder according to claim 1, wherein the bioactive compounds are essential and non-essential biological compounds obtained from foods or by-products from the agri-food industry.

3. Composition of vinegar powder according to any one of the preceding claims, wherein the bioactive compounds are selected from phenolic, aromatic, aliphatic, nitrogenous, organosulfur compounds, alkaloids, terpenes, terpenoids, fatty acids, or a combination of two or more thereof.

4. Composition of vinegar powder according to any one of the preceding claims, where the vinegar is from any product or drink of agricultural origin resulting from double alcoholic and acetic fermentation, preferably from alcohol, cider or wine.

5. Composition of vinegar powder according to any one of the preceding claims, wherein vinegar is vinegar buffered by neutralization with sodium hydroxide, sodium bicarbonate, potassium hydroxide or potassium bicarbonate.

6. Composition of vinegar powder according to any one of the preceding claims, wherein the α-cyclodextrins have the following characteristics:
- Number of glucose units: 6,
- Molecular weight: 972.
- Number of water molecules in the cavity: 6;
- Solubility in water at 25 °C: 145 g/L;
- Diameter of central cavity (nm): 0.5-0.6;
- Outer diameter (nm): 1.4-1.5; and
- Height of the toroidal shape (nm): 0.8.

7. Method for obtaining the composition of vinegar powder, according to claims 1 to 6, which includes the following steps:
a. Obtaining dehydrated and ground foods or by-products from the agri-food industry until reaching a moisture content of 3% to 12% on a wet basis and a granulometry of between 150 µm and 450 µm,
b. Obtaining vinegar of agricultural origin,
c. Completely dissolving α-CDs in the vinegar obtained in b), in a proportion of 5 to 15% (weight:volume), preferably 8 to 10%, at 22-25 °C, with stirring at 1000 to 3000 rpm, for a time 2 to 2.5 hours,
d. Mixing the ground and dehydrated food or by-product of the agri-food industry from step a), in a proportion of 1:10 to 1:60 (weight:volume), preferably in a proportion of 1:30 to 1:50, with the vinegar that includes the dissolved α-CDs obtained in c), stirred at 3000 to 3500 rpm and at a temperature of 22 to 25 °C, for 30 to 60 s,
e. Carrying out the extraction of bioactive compounds by treating the mixture obtained in d) with ultrasound at 35 kHz, at a temperature of 30 to 35 °C, for 15 to 30 minutes,
f. Filtering the solution obtained in e) through a 50 to 150 µm filter, at 22 - 25 °C, for 15 to 30 minutes,
g. Mixing the liquid extract obtained in f), after separating the solids retained in the filter, with 5-15% (weight:volume) of α-CDs, preferably 8-10%, stirred at 3000 to 4000 rpm, at 22 to 25 °C, for 15 to 30 minutes, until obtaining a homogeneous liquid, without the presence of lumps and
h. Carrying out microencapsulation by atomization of the liquid obtained in step g), in an spray dryer under the following conditions: drying air inlet temperature of 140 to 170 °C, drying air outlet temperature of 70-90 °C, pressure of the atomization compressed air of 2 to 4 bar, and liquid feed rate to be atomized of 2 to 5 L/h for every 100 m³/h of hot drying air, until obtaining a dry powder product with a particle size of 35 to 400 µm and a moisture content of 0.8 to 4%.

8. Method according to claim 7 wherein in step a) the starting point is a food selected from: dried red pepper or paprika, black pepper, nutmeg, ginger, turmeric, celery, or any other dry vegetable from which oleoresins are extracted.

9. Method for surface decontamination of solid food, which includes:
a. Directly applying the composition of vinegar powder of claims 1 to 6 on the solid food in a proportion by weight between 0.2 and 0.5%, or
b. Applying the composition of vinegar powder of claims 1 to 6, previously completely dissolved by stirring in water, in a proportion by weight equal to or less than 2%.

10. Method according to claim 9, wherein the application of the solution of the vinegar composition in b) is carried out by:
i. bathing the solid foods in the aqueous solution for a time of 0.5 to 1.5 minutes, or alternatively, by showering, at a rate of 1-1.2 liters of solution per 1000 kg of solid food, and
ii. drying and cooling the solid food treated with the aqueous solution.

11. Food product that comprises in its formulation the composition of vinegar powder of claims 1 to 6.

12. Food product, according to claim 11, **characterized in that** the common salt of the original formulation of said product is replaced with the composition of vinegar powder.

13. Food product according to any of claims 11 and 12, wherein the bioactive compounds are obtained from mushrooms or byproducts of the mushroom industry.

14. Use of the composition of vinegar powder of claims 1 to 6 for surface decontamination of food.

15. Use of the composition of vinegar powder of claims 1 to 6 in the formulation of food products.
